# FASCICULE DE BREVET EUROPEEN

(11) **EP 1 286 679 B1**
(45) Date de publication et mention de la délivrance du brevet: **04.05.2005**
(21) Numéro de dépôt: 00940480.7
(22) Date de dépôt: 09.06.2000
(51) Int. Cl.: A61K 35/78, A61K 38/43, A61P 17/00

(54) **PREPARATION A USAGE CUTANE**
HAUTPFLEGEMITTEL
SKIN TREATMENT PREPARATION

(43) Date de publication de la demande: 05.03.2003
(73) Titulaire: Life Science Investment Ltd., London SW1X 9AY (GB)
(72) Inventeur: DIEHL, Christian, 5008 CORDOBA (AR)
(74) Mandataire: Guerre, Dominique
(86) Numéro de dépôt international: PCT/FR2000/001601
(87) Numéro de publication internationale: WO 2001/093887

(56) Documents cités:
- PATENT ABSTRACTS OF JAPAN vol. 016, no. 194 (C-0938), 11 mai 1992 (1992-05-11) & JP 04 029915 A (SUNSTAR INC), 31 janvier 1992 (1992-01-31)

## Description

L'invention concerne une nouvelle préparation à usage cutané formée par un mélange d'excipients, de composés végétaux renfermant des enzymes et des vecteurs médicamenteux, destinée à prévenir ou stopper l'évolution du front inflammatoire lors des phases évolutives du vitiligo et l'éventuelle repigmentation.

Comme on le sait, l'expression 'vecteurs médicamenteux' désigne des particules creuses et de petite taille qui forment réservoirs pour des produits actifs qui se trouvent ainsi protégés et que l'on peut libérer ou relarguer le moment venu à l'endroit le plus approprié (biodisponibilité). Les liposomes, décrits par exemple dans le document FR-A-2 315 991 correspondant au document US-A-4 217 344 sont les plus connus de ces vecteurs.

Récemment, les travaux de chercheurs ont permis, à partir de la recherche fondamentale, d'avoir une meilleure connaissance du vitiligo, et d'indiquer de nouvelles directions thérapeutiques.
Ils ont examiné la théorie autosomique du vitiligo, selon la séquence suivante : la thiorédoxine-réductase, une thioenzyme présente dans les kératinocytes et les mélanocytes humains réduit les radicaux libres, fournissant ainsi possiblement la première ligne de défense contre les dommages infligés à la peau par les radicaux libres générés par les UV.
Par ailleurs, le radical anion superoxyde a été prouvé être le véritable substrat de la tyrosinase, de préférence à l'oxygène moléculaire.
Un faible taux de catalase a été relevé dans l'épiderme à la fois lésionnel et non-lésionnel de patients atteints de vitiligo, comparé à des témoins indemnes de cette maladie.
Il fut alors suggéré que ces faibles taux de catalase pouvaient conduire à une concentration plus forte de peroxyde d'hydrogène chez ces patients.
Cette augmentation du taux de H²O² pourrait induire une dépigmentation, détruire les mélanocytes, et pourrait aussi entretenir de faibles taux de catalase, H²O² pouvant inhiber le centre hématologique de la catalase.
Le peroxyde d'hydrogène peut agir comme un inhibiteur réversible de la tyrosinase. Il induit une réaction photochimique de réduction, produisant des radicaux et des ions hydroxyl hautement réactifs.
Ceux-ci sont capables de décolorer la mélanine physiologique, et d'induire la lyse membranaire des mélanocytes.
Les mêmes chercheurs ont également démontré une augmentation des TNF-alpha, GTP, 6-BH4, et PAH dans l'épiderme après exposition aux UV.
Par l'utilisation récente de la spectroscopie Raman, ils ont été en mesure de démontrer pour la première fois les niveaux élevés de H²O² dans l'épiderme de patients atteints de vitiligo.
Des études très récentes réalisées sur des cultures cellulaires de mélanocytes humains prélevés chez des malades atteints, traités par un extrait végétal riche en réductase, en catalase et en super-oxyde dismutase, ont montré de manière irrécusable la diminution du taux de peroxyde d'hydrogène sur les cellules traitées, ainsi que le recul du phénomène inflammatoire associé au vitiligo.
Ces recherches laissent donc à penser qu'une préparation cutanée renfermant ces extraits végétaux, inclus dans des vecteurs médicamenteux permettant de les protéger et d'assurer leur relargage préférentiel au niveau des kératinocytes et des mélanocytes, réduirait le profil inflammatoire associé au vitiligo de manière satisfaisante, et devrait donc limiter, voire stopper la dépigmentation cutanée.

L'invention vise donc à créer une préparation perfectionnée du type en question, qui donne les meilleurs résultats possibles sur le plan de la limitation, voire de l'arrêt de la dépigmentation cutanée, qui soit facile à fabriquer, stable, économique et ne présente pas d'effets intempestifs de relargage.

Cette préparation à usage cutané, comprenant des excipients, et un vecteur médicamenteux encapsulant un extrait végétal riche en réductase, en catalase et en super-oxyde dismutase se caractérise :
- en ce que le vecteur médicamenteux, constitué par des nanosphères encapsulant un extrait végétal riche en réductase, en catalase et en super-oxyde dismutase, représente entre 1% et 20% du poids de la préparation.
- en ce que l'extrait végétal utilisé est riche en réductase, en catalase et en super-oxyde dismutase.
Par nanosphères, on désigne des vecteurs médicamenteux constitués de particules, généralement sphériques ou ovoïdes, de taille nanométrique, encapsulant une substance déterminée, en l'occurrence un extrait végétal riche en réductase, en catalase et en super-oxyde dismutase.
Ces vecteurs sont largement décrits dans la littérature.

En d'autres termes, l'invention réside dans une double sélection, à savoir tout d'abord la sélection d'un vecteur spécifique, à savoir les nanosphères, encapsulant un actif spécifique, un extrait végétal riche en réductase, en catalase et en super-oxyde dismutase.
On ne pouvait pas penser que cette double sélection permette de résoudre avec succès le problême posé, à savoir grâce à la taille réduite des nanosphères, une meilleure pénétration jusqu'au niveau des kératinocytes et des mélanocytes et donc une meilleure biodisponibilité des actifs à ce stade, et d'autre part grâce au choix d'un extrait végétal dont le contenu en réductase, en catalase et en super-oxyde dismutase est équilibré, une efficacité satisfaisante quant à la limitation, voire à l'arrêt de la dépigmentation cutanée.

Avantageusement, en pratique :
- les nanosphères sont des sphères creuses comprenant une membrane en collagène et en glycosaminoglycannes, telles que celles commercialisées par COLETICA sous les marques déposées NANOTHALASPHERES et NANOCOLLASPHERES ; ces nanosphères sont décrites dans le document FR-A-8901221 ; ce type de vecteur présente l'avantage, en combinaison avec des excipients, de donner des préparations cutanées moins irritantes, totalement biodégradables, biocompatibles et à relargage contrôlé ;
- les nanosphères chargées en éxtrait végétal riche en réductase, catalase et superoxydant dismutase représentent entre 1% et 20% du poids de la préparation, de préférence entre 3 et 12% ; en effet on a observé que si la proportion de nanosphères est inférieure à 1%, on n'obtient pratiquement aucune limitation de la dépigmentation, et qu'en revanche lorsque cette proportion excède 20%, la libération est excessive, et susceptible de nuire aux mécanismes physiologiques normaux de la peau.
- La charge d'extrait végétal des nanosphères représente de 5 à 15% du poids de celles-ci ; on a en effet observé qu'une charge inférieure à 5% donnait des résultats inconstants sur la limitation de la dépigmentation cutanée, et l'éventuelle repigmentation tandis qu'une charge supérieure à 15% n'était industriellement pas admissible quant à la fabrication des nanosphères ; ceci dit, on obtient les meilleurs résultats avec une proportion voisine de 10%.
- L'extrait végétal retenu doit avoir les concentrations enzymatiques suivantes :
   - Réductase : entre 100 et 1.000 UI
   - Catalase : entre 100 et 1.000 UI
   - Super-oxyde dismutase : entre 500 et 5.000 UI
Des concentrations enzymatiques inférieures à 100 UI pour la réductase et la catalase et 500 UI pour la super-oxyde dismutase présentent en effet une efficacité peu satisfaisante, tandis que des concentrations supérieures à 1.000 UI pour la réductase et la catalase, et à 5.000 UI pour la super-oxyde dismutase ne sont guère compatibles avec les impératifs physiologiques de la peau humaine ; ceci dit, les meilleurs résultats ont été obtenus avec les concentrations enzymatiques suivantes :
- réductase : 500 UI
- catalase : 500 UI
- super-oxyde dismutase : 1.000 UI

La préparation contient également des excipients d'usage courant pour une telle application cutanée.
Ces excipients ne doivent pas affecter la stabilité des nanoparticules, ni interréagir avec celles-ci, ni entre eux. Il est donc préférable que la proportion de corps gras soit aussi réduite que possible, et notamment inférieure à la moitié, voire au tiers. De manière usuelle, le mélange d'excipients peut aussi contenir des agents anti-oxydants et/ou des agents conservateurs connus pour une telle préparation.

Dans une forme de réalisation pratique, la préparation selon l'invention comporte :
- 10% de nanosphères encapsulant l'actif végétal
- 1% d'agent conservateur
le reste à 100% étant constitué par de l'eau déminéralisée et un mélange d'excipients d'usage courant pour une application cutanée.

Il va de soi que dans la préparation selon l'invention, il est indispensable que les nanosphères encapsulant l'extrait végétal et les excipients ne réagissent pas entre eux.

Généralement, la préparation finie est de couleur très claire, voire beige clair, selon la qualité des corps gras auxquels on fait appel. Cette préparation est onctueuse, lisse, facile à étaler, se conserve aisément à température ambiante, notamment dans un tube exempt d'air, même au-delà d'une année.

Cette préparation cutanée s'applique aisément à la main, et pénètre rapidement sous l'effet d'un léger massage.
On obtient de bons résultats en appliquant cette préparation deux fois par jour pendant un à cinq mois, voire de façon continue.
Une fois la préparation appliquée sur la peau, les nanosphères encapsulant l'extrait végétal pénètrent dans le stratum corneum et viennent en contact avec les kératinocytes et les mélanocytes. Les deux membranes fusionnent alors, ce qui fait que l'extrait végétal passe dans le cytoplasme du kératinocyte et du mélanocyte, y entraînant les réductase, catalase et super-oxyde dismutase qu'il contient.
Il s'ensuit une réaction chimique de réduction du peroxyde d'hydrogène qui s'y trouve en quantité excessive, et une perte réduite, voire annulée en mélanine. Il s'ensuit une limitation, voire un arrêt de la dépigmentation liée au vitiligo et l'éventuelle repigmentation. Il s'ensuit également une diminution, voire une disparition du front inflammatoire associé bien souvent aux dépigmentations, phénomènes qu'on ne savait pas obtenir jusqu'alors.
La manière dont l'invention peut être réalisée et les avantages qui en découlent, ressortiront mieux des exemples de réalisation qui suivent.

### Exemple 1

A température ambiante, et sous légère agitation, dans 220 grammes (22%) d'eau déminéralisée, on mélange respectivement :
- 3 grammes (0,30%) d'un agent gélifiant à base de cellulose modifiée commercialisée par GOODRICH sous la marque 'CARBOPOL 934'
- 30 grammes (3%) de nanosphères encapsulant un extrait végétal riche en réductase, catalase et super-oxyde dismutase, se présentant sous forme d'une suspension aqueuse et commercialisée par COLETICA sous la marque 'NANOTHALASPHERES', et dont la membrane est en collagène et en glycosaminoglycannes ; ces nanosphères sont présentées comme étant des microcapsules de diamètre inférieur à un (1) micromètre, caractérisées en ce qu'elles comprennent une paroi mixte de collagène marin ou d'atélocollagène, et de glycosaminogly-cannes réticulés, la proportion des glycosaminoglycannes par rapport au collagène marin ou à l'atélocollagène pouvant varier de 15% à 50% en poids ; les glycosaminoglycannes sont choisis parmi le groupe consistant du chondroïtine-4-sulfate, du chondroïtine-6-sulfate du dermatane-sulfate, de l'héparine-sulfate, du kératane-sulfate et de l''éparine et ses dérivés.;
- 10 grammes (1%) d'un agent conservateur liquide à base de propylèneglycol, de diazolidinylurée, de méthylparabèn et de propylparabèn, commercialisé par SUTTON sous la marque 'GERMABEN II' ;
- 1 gramme (0,1%) d'un agent chélateur d'ions, connu sous le nom d'EDTA disodique ;
On agite lentement à température ambiante, jusqu' à obtenir un mélange bien homogène.

Séparément, sous agitation rapide (1.000 tours / minute), et à 80°C, on mélange dans l'ordre :
- 10 grammes (1%) d'un excipient à base d'alcool cétylique ;
- 90 grammes (9%) d'un autre excipient à base de myristate de 2-octyldodécyle ;
- 80 grammes (8%) d'un excipient à base d'un dérivé non ionique de cire d'abeille blanche, commercialisé par GATTEFOSSE sous la marque 'APIFIL' ;
- 80 grammes (8%) d'un excipient à base d'isostéarate d'isostéaryle.

On agite rapidement jusqu'à obtenir une parfaite homogénéisation.
On ajoute alors dans ce dernier mélange de l'eau déminéralisée, chauffée à 82°C, à raison de 456,5 grammes (45,65%). Pour amorcer l'émulsion, on agite rapidement, en versant cette eau dans le mélange.
Lorsque l'émulsion est formée, et que la température est redescendue à 30-35°C, on ajoute alors la première phase contenant les nanosphères d'actif végétal, puis on ajoute 6 grammes (0,6%) d'une solution à 50% de triéthanolamine pour ajuster le pH, puis 3 grammes (0,3%) d'un parfum et enfin 0,5 gramme (0,05%) d'un agent anti-oxydant commercialisé par GATTEFOSSE sous la référence 'WL 774'.

On mélange l'ensemble lentement à 30-35°C.

La préparation obtenue est une émulsion de type huile dans eau, de couleur blanche-beige clair (suivant la qualité des corps gras utilisés), lisse, onctueuse, semi-liquide, dont le pH est compris entre 5 et 7,5. Son odeur *sui generi* est liée au parfum utilisé.

Cette préparation stable dans le temps (pas de relargage intempestif après un an de stockage) peut être appliquée sur la peau à raison de deux applications par jour pendant une période illimitée.
On obtient, quelquefois dès la troisième semaine, plus généralement dans les cinq mois suivant le début de l'application, une limitation, voire un arrêt de la dépigmentation cutanée liée au vitiligo et l'éventuelle repigmentation.
De plus, l'application de cette émulsion semble prévenir de nouvelles dépigmentations.

### Exemple 2

On répète l'exemple 1 en supprimant la nanosphère encapsulant l'extrait végétal. On n'observe aucun effet.

### Exemple 3

On répète l'exemple 1 en remplaçant les nanosphères par des liposomes du commerce encapsulant l'extrait végétal.

On obtient une émulsion ayant sensiblement la même apparence et les mêmes propriétés physiques qu'à l'exemple 1. Toutefois, on observe une limitation de la dépigmentation plus lentement, et de manière plus sporadique que dans l'exemple 1.

### Exemple 4

On répète l'exemple 1 en remplaçant l'extrait végétal choisi par un autre extrait végétal ne contenant ni réductase, ni catalase, ni super-oxyde dismutase. On n'observe aucun résultat.

Ces exemples montrent bien l'activité liée à l'effet de synergie résultant de la combinaison des nanosphères encapsulant un extrait végétal riche en réductase, catalase et super-oxyde dismutase sur la limitation de la dépigmentation et la réduction du front inflammatoire au cours du processus de développement du vitiligo et l'éventuelle repigmentation.

De la sorte, ces préparations cutanées peuvent être utilisées avec succès chez des patients présentant une pathologie évolutive de vitiligo.

## Revendications

1. Préparation à usage cutané, comprenant des excipients et un vecteur médicamenteux encapsulant un extrait végétal riche en réductase, catalase et super-oxyde dismutase, **caractérisée :**
- **en ce que** le vecteur médicamenteux, constitué par des nanosphères encapsulant un extrait végétal riche en réductase, en catalase et en super-oxyde dismutase, représente entre 1% et 20% du poids de la préparation.
- **en ce que** l'extrait végétal utilisé est riche en réductase, en catalase et en super-oxyde dismutase.

2. Préparation à usage cutané selon la revendication 1, **caractérisée en ce que** les nanosphères en forme de particules sphériques encapsulant l'extrait végétal ont une membrane en collagène et en glycosaminoglycanne.

3. Préparation à usage cutané selon l'une des revendications 1 et 2, **caractérisée en ce que** la charge d'extrait végétal des nanosphères représente de 5% à 15% du poids de celles-ci.

4. Préparation à usage cutané selon la revendication 1, **caractérisée en ce que** l'extrait végétal retenu doit avoir les concentrations enzymatiques suivantes :
• réductase : entre 100 UI et 1.000 UI
• catalase : entre 100UI et 1.000 UI
• super-oxyde dismutase : entre 500 UI et 5.000 UI

5. Préparation à usage cutané selon l'une des revendications 1 à 4, **caractérisée en ce que** les excipients contiennent un mélange d'huiles et d'acides gras, des agents anti-oxydants et des agents conservateurs.

6. Préparation à usage cutané comprenant des excipients, des vecteurs médicamenteux encapsulant un extrait végétal riche en réductase, catalase et super-oxyde dismutase, **caractérisée en ce qu'**elle contient pour cent parties :
- 1 à 20%, de préférence 3%, de nanosphères encapsulant un extrait végétal riche en réductase, catalase et super-oxyde dismutase ;
- 1% d'agent conservateur ;
- 0,1% d'un agent chélateur d' ions ;
- 0,3% d'un agent gélifiant à base de cellulose modifiée ;
- 0,6% d'un agent pour ajuster le pH ;
le reste à 100% étant constitué par de l'eau déminéralisée, et un mélange d'excipients d'usage courant pour une application cutanée.

## Patentansprüche

1. Dermatologische Zusammensetzung, die Grundstoffe und einen medizinischen Vektor, in den ein reduktase-, katalase- und superoxiddismutasereicher Pflanzenextrakt eingekapselt ist, umfaßt, **dadurch gekennzeichnet, daß**
- der medizinische Vektor, der aus Nanosphären, in denen ein reduktase-, katalase- und superoxiddismutasereicher Pflanzenextrakt eingekapselt ist, besteht, zwischen 1% und 20% des Gewichts der Zusammensetzung ausmacht, und daß
- der verwendete Pflanzenextrakt, reduktase-, katalase- und superoxiddismutasereich ist.

2. Dermatologische Zusammensetzung nach Anspruch 1, **dadurch gekennzeichnet, daß** die Nanosphären, die in Form von hohlkugelartigen Partikeln, in denen der Pflanzenextrakt eingekapselt ist, vorliegen, eine Membran aus Kollagen und Glycosaminoglycan aufweisen.

3. Dermatologische Zusammensetzung nach Anspruch 1 oder 2, **dadurch gekennzeichnet, daß** der Gehalt der Nanosphären an Pflanzenextrakt 5% bis 15% des Gewichts der Nanosphären beträgt.

4. Dermatologische Zusammensetzung nach Anspruch 1, **dadurch gekennzeichnet, daß** der erhaltene Pflanzenextrakt die folgenden Enzymkonzentrationen aufweisen muß:
• Reduktase: zwischen 100 UI und 1.000 UI
• Katalase: zwischen 100 UI und 1.000 UI
• Superoxiddismutase: zwischen 500 UI und 5.000 UI

5. Dermatologische Zusammensetzung nach einem der Ansprüche 1 bis 4, **dadurch gekennzeichnet, daß** die Grundstoffe eine Mischung aus Ölen und Fettsäuren, Antioxidantien und Konservierungsstoffen enthalten.

6. Dermatologische Zusammensetzung, die Grundstoffe, medizinische Vektoren, in denen ein reduktase-, katalase- und superoxiddismutasereicher Pflanzenextrakt eingekapselt ist, enthält, **dadurch gekennzeichnet, daß** sie pro hundert Teile:
- 1 bis 20%, vorzugsweise 3%, Nanosphären in denen ein reduktase-, katalase- und superoxiddismutasereicher Pflanzenextrakt eingekapselt ist,
- 1% Konservierungsstoff,
- 0,1% Ionen-Chelatisierungsmittel,
- 0,3% Geliermittel auf Grundlage von modifizierter Zellulose,
- 0,6% eines pH-Regulators
enthält, wobei der Rest auf 100% aus entmineralisiertem Wasser und einer Mischung von Grundstoffen, die üblicherweise für dermatologische Zwecke verwendet werden, besteht.

## Claims

1. Skin treatment preparation, comprising excipients and a medicinal vector encapsulating a plant extract rich in reductase, catalase and superoxide dismutase, **characterized:**
- **in that** the medicinal vector, consisting of nanospheres encapsulating a plant extract rich in reductase, catalase and superoxide dismutase, represents between 1% and 20% of the weight of the preparation,
- **in that** the plant extract used is rich in reductase, catalase and superoxide dismutase.

2. Skin treatment preparation according to Claim 1, **characterized in that** the nanospheres in the form of spherical particles encapsulating the plant extract have a membrane made of collagen and glycosaminoglycan.

3. Skin treatment preparation according to either of Claims 1 and 2, **characterized in that** the plant extract load of the nanospheres represents from 5% to 15% of the weight of these nanospheres.

4. Skin treatment preparation according to Claim 1, **characterized in that** the plant extract selected should have the following enzyme concentrations:
• reductase: between 100 IU and 1000 IU
• catalase: between 100 IU and 1000 IU
• superoxide dismutase: between 500 IU and 5000 IU.

5. Skin treatment preparation according to one of Claims 1 to 4, **characterized in that** the excipients contain a mixture of oils and fatty acids, antioxidants and preservatives.

6. Skin treatment preparation comprising excipients and medicinal vectors encapsulating a plant extract rich in reductase, catalase and superoxide dismutase, **characterized in that** it contains, per hundred parts:
- 1 to 20%, preferably 3%, of nanospheres encapsulating a plant extract rich in reductase, catalase and superoxide dismutase;
- 1% of preservative;
- 0.1% of an ion-chelating agent;
- 0.3% of a gelling agent based on modified cellulose;
- 0.6% of an agent for adjusting the pH;
the remainder up to 100% consisting of demineralized water and a mixture of excipients commonly used for a skin application.
